# DEMANDE DE BREVET EUROPEEN

(11) **EP 2 196 929 A1**
(43) Date de publication de la demande: **16.06.2010**
(21) Numéro de dépôt: 09178520.4
(22) Date de dépôt: 09.12.2009
(51) Int. Cl.: G06F 19/00

(54) **Système de gestion d'informations relatives à un animal de compagnie portant des moyens électroniques d'identification sous-cutanés**

(30) Priorité: 10.12.2008 FR 0858454
(71) Demandeur: Allflex Europe, 35500 Vitre (FR)
(72) Inventeur: De Meulemeester, Johan, 9840 De Pinte (BE); Boiron, Dominique, 35500 Vitre (FR)
(74) Mandataire: Bioret, Ludovic

(57) **Abrégé**

L'invention concerne un système de gestion d'informations relatives à au moins un animal de compagnie portant des moyens électroniques d'identification (10) sous-cutanés, un tel système comprenant :
- des moyens de lecture (11) d'une information d'identification contenue dans lesdits moyens électroniques d'identification ;
- des moyens de mesure (12) d'au moins une donnée physique associée audit animal ;
- des moyens de stockage et/ou transmission (13) de ladite au moins une donnée physique, en tenant compte de ladite information d'identification.

Selon l'invention, lesdits moyens de mesure (12) comprennent au moins une balance (20) et lesdits moyens de lecture (11) sont intégrés à ladite balance (20).

## Description

### 1. Domaine de l'invention

Le domaine de l'invention est celui de l'identification et du suivi des animaux.

Plus précisément, l'invention concerne la gestion d'informations relatives à un animal de compagnie portant un implant sous-cutané comprenant des moyens électroniques d'identification.

### 2. Art antérieur

L'identification des animaux domestiques ou de compagnie a été rendue obligatoire dans de nombreux pays. Ainsi, en France, les animaux de compagnie comme les chiens ou les chats doivent pouvoir être identifiés par un tatouage ou une puce électronique depuis plusieurs années. Cette identification permet à l'animal d'être répertorié dans un fichier, dans lequel figurent les coordonnées de son propriétaire.

On constate ces dernières années un développement des moyens électroniques d'identification des animaux, et notamment des puces électroniques destinées à être implantées sous la peau d'un animal de compagnie. Classiquement, une puce électronique est implantée dans le cou de l'animal. De telles puces présentent de nombreux avantages, comme une pose facile et sans douleur, une identification fiable des animaux puisque ces puces restent lisibles durant toute la vie de l'animal et sont théoriquement infalsifiables, etc.

Ces puces se présentent sous la forme d'un transpondeur comprenant généralement une bobine associée à un élément électronique intégrant des fonctions de communication et de stockage de données, encapsulés dans une ampoule en verre. L'élément électronique porte notamment un numéro d'identification de l'animal, ou identifiant, permettant de le suivre tout au long de son existence.

De tels transpondeurs sont notamment décrits dans les brevets américains US 5,025,550 et US 5,281,855.

Actuellement, ces moyens d'identification des animaux de compagnie, qu'ils soient électroniques ou non, ne sont utilisés que pour assurer le suivi statistique et administratif des animaux et pour permettre d'identifier leurs propriétaires.

### 3. Exposé de l'invention

L'invention propose une nouvelle application des moyens électroniques d'identification, et se présente sous la forme d'un système de gestion d'informations relatives à au moins un animal de compagnie portant des moyens électroniques d'identification sous-cutanés, un tel système comprenant :
- des moyens de lecture d'une information d'identification contenue dans les moyens électroniques d'identification ;
- des moyens de mesure d'au moins une donnée physique associée à l' animal ;
- des moyens de stockage et/ou transmission de la ou des donnée(s) physique(s), en tenant compte de l'information d'identification.

Selon l'invention, les moyens de mesure comprennent au moins une balance et les moyens de lecture sont intégrés à la balance.

L'invention propose ainsi une nouvelle application des moyens électroniques d'identification, permettant d'assurer le suivi « physique » des animaux domestiques ou de compagnie. On entend ici par animal de compagnie tout animal détenu ou destiné à être détenu par l'homme pour son agrément.

Pour ce faire, l'invention propose un système permettant de mesurer une ou plusieurs données physiques (ou physiologique, morphologique, etc) associées à l'animal, comme son poids, sa température, sa taille, etc, et d'associer automatiquement ces données mesurées à l'identifiant de l'animal.

Un tel système comprend au moins une balance rendant ainsi possible la mesure du poids de l'animal, et le suivi de l'évolution de ce poids tout au long de l'existence de l'animal.

En outre, les moyens de lecture sont intégrés ou solidarisés à la balance.

Ainsi, le seul fait de positionner l'animal sur la balance permet :
- de mesurer au moins une donnée physique associée à l'animal, ici son poids ; et
- de lire une information d'identification contenue dans les moyens électroniques d'identification.

Ces informations d'identification et de poids peuvent être stockées dans une mémoire, ou transmise à un équipement distant.

Cette association est mise en oeuvre automatiquement, c'est-à-dire sans intervention extérieure, comme l'intervention d'un vétérinaire ou du propriétaire de l'animal par exemple. Un tel système fonctionne donc de manière autonome.

Il est ensuite possible de stocker les données mesurées associées à l'identifiant de l'animal dans une mémoire, par exemple sous la forme d'une liste, comprenant pour un ou plusieurs animaux :
- une information d'identification de l'animal (numéro d'identification par exemple) ;
- une ou plusieurs données physiques liée(s) à cette information d'identification.

On note que ces données physiques peuvent être mesurées à différentes dates. Selon cet aspect, le système selon l'invention comprend des moyens d'association d'une date à au moins une donnée physique.

Il est également possible de transmettre les données mesurées à un équipement distant, éventuellement après enregistrement de ces données. Par exemple, ces données peuvent être envoyées au vétérinaire en charge de l'animal ou au propriétaire de l'animal, sur leurs ordinateurs. L'information d'identification peut être transmise conjointement à ces données, ou être utilisée uniquement pour identifier le propriétaire de l'animal et lui transmettre ces données (sur sa messagerie électronique par exemple).

Selon un aspect particulier de l'invention, les moyens de lecture comprennent au moins une antenne de type antenne cadre bobinée.

Par exemple, une telle antenne peut être non plane de manière à couvrir différentes orientations des moyens électroniques d'identification situés dans le champ de l'antenne.

L'antenne peut être mobile autour d'un axe de la balance, afin d'améliorer la lecture des moyens électroniques d'identification.

Selon une variante, les moyens de lecture sont intégrés à un lecteur amovible. Ce lecteur peut communiquer avec les moyens de stockage et/ou transmission par une liaison filaire ou sans fil. Il est par exemple intégré à une souris d'ordinateur.

Cette variante nécessite qu'un utilisateur, comme le propriétaire de l'animal par exemple, « scanne » la puce électronique de l'animal. Ensuite, les données mesurées sont automatiquement associées à l'identifiant de l'animal.

Selon une caractéristique particulière, le système de gestion d'informations comprend au moins un processeur de traitement, permettant de relier la ou les donnée(s) physique(s) mesurées à l'information d'identification.

Un tel processeur est piloté par un produit programme d'ordinateur permettant de relier l'information d'identification à différentes informations, comme les données physiques mesurées, le nom de l'animal ou du propriétaire, un historique de poids, etc.

Il peut être intégré dans les moyens de mesure, comme dans une balance par exemple. Il peut communiquer avec une mémoire stockant les données mesurées, éventuellement associées à une date et à un numéro d'identification. Il peut également communiquer avec des moyens de transmission de la ou des données mesurées.

En particulier, un tel processeur peut délivrer une information de comparaison entre au moins une donnée physique mesurée précédemment et une donnée physique courante, associées à l'animal.

Par exemple, le processeur peut comparer le poids de l'animal mesuré à un jour donné avec le poids du même animal mesuré antérieurement et stocké dans une mémoire (présente dans la balance par exemple, ou dans un équipement distant).

Le processeur peut alors délivrer au moins une recommandation en fonction d'une analyse de cette information de comparaison. Par exemple, si l'animal a pris du poids, et est en surpoids par rapport à une courbe de référence, une recommandation relative à l'alimentation de l'animal peut être délivrée, comme une quantité de nourriture par jour, un type de croquettes particulier, etc.

En particulier, le système de gestion d'informations peut comprendre au moins un haut-parleur. Le système peut également être équipé d'un système de synthèse vocale, permettant de personnaliser le message vocal.

Par exemple, si l'animal est un chien prénommé « Médor », le système peut énoncer un message du type « Médor pèse aujourd'hui *x* kg ».

De la même façon, la recommandation peut être émise sous forme orale. Par exemple « Médor a pris *y* kg depuis la pesée du *d*. Nous recommandons l'utilisation de croquettes allégées ».

Selon un mode de réalisation particulier de l'invention, le système de gestion d'informations comprend des moyens d'affichage et/ou de saisie d'informations liées à l'animal.

Par exemple, ces moyens d'affichage et/ou de saisie comprennent au moins un élément appartenant au groupe comprenant :
- un écran d'affichage ;
- un écran tactile ;
- un clavier de programmation.

Les moyens d'affichage permettent notamment d'afficher des messages publicitaires ou d'informations.

Selon un aspect particulier de l'invention, les moyens de stockage et/ou transmission du système de gestion d'informations coopèrent avec au moins une connexion permettant un transfert d'au moins la ou les donnée(s) physique(s) mesurées vers une unité externe.

Cette unité externe, également appelée équipement distant, correspond par exemple à l'ordinateur du vétérinaire, de son assistant, ou du propriétaire, ou un terminal de communication du propriétaire de type téléphone portable ou assistant personnel de type PDA (en anglais « Personal Digital Assistant »), etc.

Par exemple, la connexion appartient au groupe comprenant :
- une connexion USB (en anglais « Universal Serial Bus », en français « bus série universel) ;
- une connexion série ;
- une connexion Ethernet ;
- une connexion Bluetooth (marque déposée) ;
- une connexion Wifi.

De cette façon, il est possible de connecter une clé USB (à la balance par exemple) pour récupérer les informations stockées dans les moyens de stockage. Il est également possible de communiquer à distance avec la balance.

L'invention concerne également une balance équipée de moyens de lecture d'une information d'identification contenue dans des moyens électroniques d'identification équipant un animal de compagnie.

### 4. Liste des figures

D'autres caractéristiques et avantages de l'invention apparaîtront plus clairement à la lecture de la description suivante d'un mode de réalisation particulier, donné à titre de simple exemple illustratif et non limitatif, et des dessins annexés, parmi lesquels :
- la figure 1 illustre le principe général d'un système de gestion d'informations relatives à un animal de compagnie portant une puce électronique selon un mode de réalisation particulier de l'invention ;
- les figures 2A et 2B présentent deux structures d'antennes pouvant être utilisées pour lire l'information d'identification de l'animal stockée dans la puce électronique ;
- la figure 3 illustre les interactions entre le système selon un mode de réalisation particulier de l'invention et des équipements distants.

### 5. Description d'un mode de réalisation de l'invention

Le principe général de l'invention repose sur l'association automatique d'une ou plusieurs données physiques d'un animal domestique ou de compagnie, à une information d'identification de l'animal contenue dans des moyens électroniques d'identification sous-cutanés, appelés communément puce électronique.

L'invention propose ainsi une nouvelle application des moyens électroniques d'identification, permettant d'assurer le suivi « physique » (ou physiologique, morphologique, etc) de ces animaux, comme le suivi de leur poids, leur taille, leur température, etc.

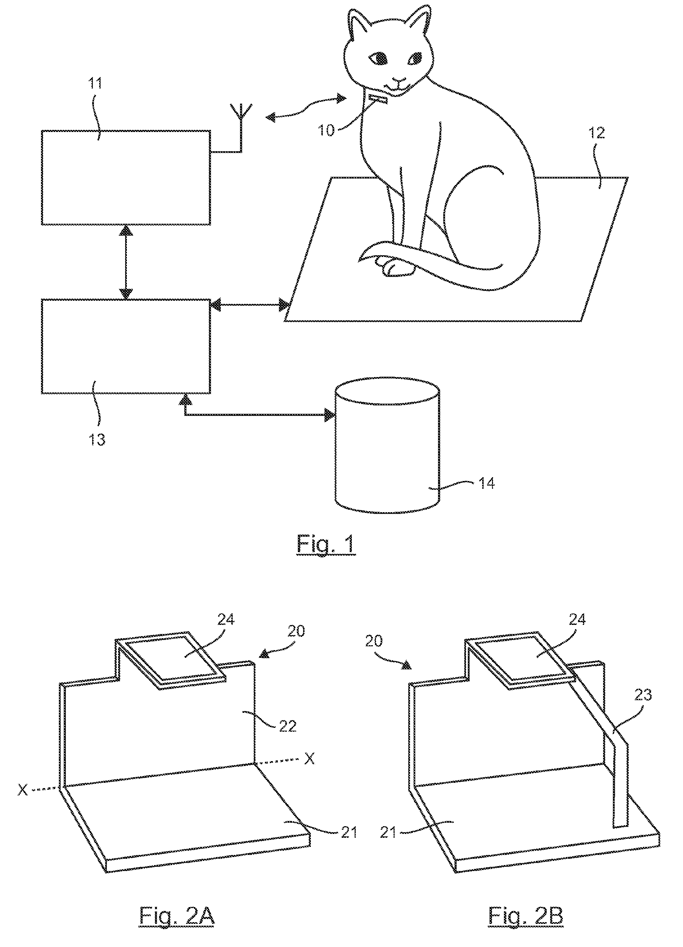

Pour ce faire, l'invention propose un système de gestion d'informations relatives à un ou plusieurs animaux tel qu'illustré en figure 1. Un tel système comprend :
- des moyens de lecture 11 d'une information d'identification contenue dans des moyens électroniques d'identification 10 implantés sous la peau d'un animal (par exemple de type RFID, en anglais « radio frequency identification », en français « identification par radiofréquence ») ;
- des moyens de mesure 12 d'au moins une donnée physique associée à l' animal ;
- des moyens de stockage et/ou transmission 13 de la ou des donnée(s) physique(s) mesurée(s), en tenant compte de l'information d'identification.

Les données mesurées peuvent être stockées dans une base de données 14, mettant en correspondance les données mesurées et l'information d'identification de l'animal correspondant, et éventuellement la date du jour. Il est ainsi possible de suivre l'évolution de l'animal, par son poids ou sa taille par exemple.

On décrit ci-après un exemple particulier de l'invention, selon lequel les moyens de mesure d'au moins une donnée physique associée à l'animal (chat ou chien par exemple) comprennent une balance comportant un plateau de pesage. On mesure donc le poids de l'animal.

Bien entendu, cet exemple est purement illustratif, et non limitatif, et d'autres caractéristiques physiques de l'animal, comme sa taille, sa température, etc, pourraient être mesurés.

Ainsi qu'illustré en figure 2A et 2B, les moyens de lecture comprennent au moins une antenne associée à un plateau de pesage 21.

Plus précisément, selon la figure 2A, l'antenne peut être intégrée dans une paroi 22 de la balance 20, et prendre la forme d'une antenne cadre bobinée, ou d'une boucle de fil. Son diagramme est alors perpendiculaire au plan de la boucle, afin de pouvoir récupérer les informations présentes dans une puce électronique implantée sous la peau d'un animal présent sur le plateau de pesage 21.

Selon une variante illustrée en figure 2B, l'antenne prend la forme d'un bras 23, pliable autour de l'animal, permettant d'améliorer la lecture des moyens électroniques d'identification. En effet, l'utilisation d'une antenne non plane permet de couvrir différentes orientations de la puce électronique située dans le champ de l'antenne. Par exemple, si une telle puce prend la forme d'un cylindre, il est possible de lire les informations qu'elle porte quelle que soit l'orientation du cylindre.

Selon une autre variante, l'antenne peut être mobile autour d'un axe de la balance. Ceci permet d'améliorer la lecture de la puce électronique, notamment en rapprochant l'antenne de l'animal. Par exemple, la paroi 22 est mobile par rapport au plateau de pesage 21, autour de l'axe XX situé à l'intersection de la paroi 22 et du plateau de pesage 21.

L'antenne peut également être une antenne non plane intégrée dans la paroi 22 et l'élément 24. L'angle entre la paroi 22 et l'élément 24 peut alors être adapté pour tenir compte de la mobilité de la paroi 22.

D'autres types ou géométries d'antennes peuvent également être envisagés, du moment qu'ils permettent la lecture (ou la réception) d'informations portées par des moyens électroniques d'identification sous-cutanés.

En d'autres termes, l'antenne du système est positionnée pour optimiser la lecture d'un implant électronique sous-cutané ou intramusculaire chez des animaux domestiques ou de compagnie.

Une balance 20 selon les figures 2A ou 2B peut par exemple être déplacée et installée dans la salle d'attente ou de consultation d'un vétérinaire, dans une pharmacie, dans un centre commercial, etc, pour assurer un suivi du poids de l'animal notamment. Les dimensions de la balance 20 sont limitées aux animaux domestiques ou de compagnie, la balance 20 étant apte à recevoir un animal dont le poids maximal est compris entre 150 et 200 kg par exemple.

Par exemple, lorsque le propriétaire d'un animal amène celui-ci chez le vétérinaire pour se faire vacciner, le propriétaire de l'animal peut utiliser ce service pour contrôler le poids de son animal, en attendant sa prise en charge par le vétérinaire.

Pour se faire, il suffit que l'animal soit placé sur le plateau de pesage 21 pour être pesé. L'antenne comprise dans la paroi 22 et/ou l'antenne pliable 23 reçoit alors (ou lit) une information d'identification de l'animal, contenue dans la puce sous-cutanée de l'animal.

Le poids de l'animal est alors stocké et/ou transmis, en tenant compte de l'information d'identification. Pour ce faire, la balance 20 comprend au moins une connexion (ou un port) permettant un transfert vers une unité externe et/ou une mémoire. Par exemple, le poids de l'animal est stocké dans la base de données 14, en relation avec la date du jour et le numéro d'identification de l'animal. Il est également possible de transmettre le poids mesuré au domicile du propriétaire de l'animal, sur son ordinateur par exemple, en utilisant l'information d'identification pour identifier le propriétaire de l'animal et son adresse de courrier électronique par exemple.

La balance 20 dite « intelligente » comprend ainsi au moins un processeur, piloté par un produit programme d'ordinateur permettant de relier un numéro d'identification de l'animal à différentes informations, comme le nom de l'animal ou de son propriétaire, la donnée courante mesurée, au moins une donnée mesurée précédemment, etc.

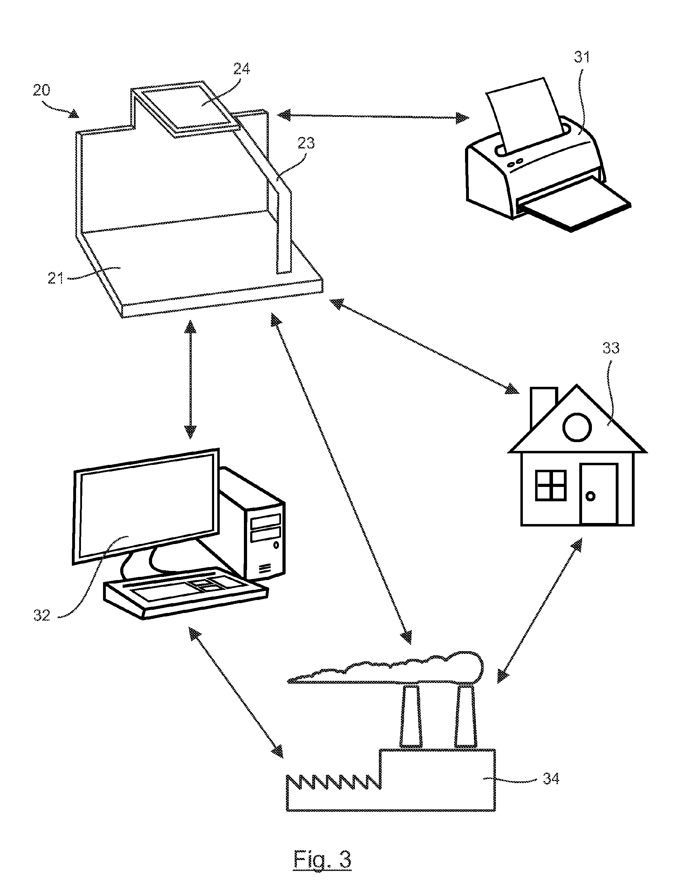

Comme illustré en figure 3, une imprimante 31 peut également être connectée la balance 20, ou intégrée dans la balance 20. De cette façon, le propriétaire peut imprimer un graphique montrant l'évolution du poids (ou de la taille, température, etc) de l'animal au cours du temps, en tenant compte des données associées à l'animal précédemment mémorisées dans la base de données 14.

D'autres connexions sont possibles, comme une connexion USB, série, Ethernet, Bluetooth (marque déposée), Wifi, etc.

Ainsi, le propriétaire de l'animal peut charger les données mesurées sur une clé USB par exemple.

Selon une variante, la balance 20 peut également communiquer (par exemple par une liaison sans fil de type Bluetooth) avec l'ordinateur 32 du vétérinaire, qui peut réaliser le suivi de l'animal, ou avec un terminal du propriétaire, comme son téléphone portable ou son PDA.

Si les données mesurées sont communiquées à l'ordinateur 32 du vétérinaire, ces données peuvent être accessibles pour le propriétaire par l'intermédiaire du site Internet du vétérinaire, via le numéro d'identification présent dans la puce de l'animal par exemple.

A l'inverse, si le propriétaire de l'animal dispose d'une telle balance à son domicile 33, les données peuvent être transférées au vétérinaire.

Selon l'exemple particulier décrit, la balance 20 comprend également un haut-parleur, et des moyens de synthèse vocale.

Ainsi, la balance 20 peut reconnaître l'animal grâce à sa puce, et communiquer avec le propriétaire de façon personnalisée. Par exemple, une fois l'animal pesé et reconnu, la balance peut émettre un message vocal du type « Bonjour Monsieur (nom du propriétaire), le poids corporel de (nom de l'animal) aujourd'hui est de 5,650 kg, soit une diminution de 600 g par rapport à la dernière visite du (date de la dernière visite) ».

La balance peut également être équipée d'un écran 24, par exemple un écran tactile, permettant notamment d'afficher le poids de l'animal.

Par exemple, cet écran tactile (ou un clavier classique) peut être utilisé pour entrer le nom du propriétaire et de son animal, l'adresse électronique du propriétaire, etc, en correspondance avec l'identifiant de l'animal porté par la puce électronique. Ces informations peuvent être mémorisées dans la base de données 14, en relation avec l'information d'identification contenue dans la puce électronique par exemple.

Cet écran 24 peut également être dissocié de la balance 20, et communiquer avec la balance 20 via une liaison filaire ou sans fil.

De plus, il est possible d'afficher sur l'écran 24 des publicités, ou des recommandations à destination du propriétaire de l'animal par exemple. Les publicités proviennent par exemple de firmes pharmaceutiques ou de distributeurs de nourriture pour animaux 34, et peuvent être mises à jour régulièrement par ces firmes ou distributeurs 34. Elles peuvent évoluer en fonction des saisons (en proposant des produits pour lutter contre les puces ou les tiques en été ou en automne), en fonction de la visite d'un représentant, et peuvent être ciblées en fonction de l'animal ou du suivi de l'animal (proposition de croquettes allégées spécial chat si l'animal est un chat en surpoids par exemple, ou d'un produit vermifuge si le chat a perdu du poids). Elles peuvent également proposer des exercices adaptés à l'animal, des conseils sur le régime alimentaire, etc.

Les firmes pharmaceutiques ou les distributeurs de nourriture 34 peuvent également communiquer avec l'ordinateur du vétérinaire 32, par exemple par une liaison Internet, notamment lorsque l'ordinateur 32 centralise les informations relatives aux animaux. Par exemple, il est possible d'effectuer des statistiques sur le poids des animaux et leur éventuelle surcharge, et de communiquer ces informations aux firmes ou aux distributeurs de nourriture 34.

Les firmes ou les distributeurs de nourriture 34 peuvent alors proposer au vétérinaire, sur son ordinateur 32, des promotions ciblées en fonction du profil des animaux, de nouveaux produits alimentaires ou médicamenteux, etc.

Il est également possible d'afficher sur cet écran le poids mesuré de l'animal et le poids de référence d'un animal de même sexe, même race, etc, afin que le propriétaire de l'animal puisse contrôler que son animal se situe dans la moyenne. Ces informations de poids idéal par rapport à la race de l'animal peuvent également être imprimées via l'imprimante 31. Il est aussi possible d'afficher un poids cible que l'animal devrait atteindre (entré par le propriétaire ou déterminé en fonction des courbes de poids idéal), et des recommandations pour que l'animal atteigne ce poids cible (sous forme d'une série d'exercices, de recommandations alimentaires, etc, proposées et mises à jour par les firmes ou les distributeurs de nourriture 34).

D'autres informations personnalisées liées à l'information d'identification de la puce électronique de l'animal peuvent également s'afficher.

Ainsi, le système selon l'invention est particulièrement intéressant du fait de son autonomie, dans le sens où aucune intervention extérieure, comme l'intervention d'un vétérinaire ou du propriétaire de l'animal, n'est nécessaire. Les frais de personnel pour le vétérinaire sont donc diminués lorsque le système est installé dans la salle d'attente du vétérinaire.

Bien que dans l'exemple décrit les différents éléments de l'invention soient intégrés à la balance, cet exemple n'est pas limitatif.

Par exemple, les moyens de lecture peuvent être amovibles, et peuvent communiquer avec le processeur et/ou une mémoire et/ou la base de données 14 par une liaison filaire ou sans fil. En d'autres termes, l'antenne 23 n'est pas nécessairement intégrée à la balance, mais peut être déportée.

De la même façon, les moyens de stockage des données physiques peuvent être présents dans la balance sous la forme d'une mémoire, ou déportés dans l'ordinateur 32 du vétérinaire ou un terminal du propriétaire. Il peut également s'agir d'un moyen de stockage amovible de type clé USB.

Selon une variante, les moyens de lecture prennent la forme d'un lecteur portatif, qui transmet l'information d'identification contenue dans les moyens électroniques d'identification à un processeur de traitement.

Un tel lecteur portatif est par exemple utilisé par le propriétaire de l'animal à son domicile 33. Il peut ainsi communiquer avec l'ordinateur du propriétaire de l'animal. Par exemple, ce lecteur est intégré à la souris de l'ordinateur du propriétaire. Selon une variante, ce lecteur est un lecteur indépendant, qui peut être rechargé ou transmettre les informations qu'il a lu lorsqu'il est posé sur un support connecté à un port USB de l'ordinateur.

Un tel lecteur, même s'il ne communique pas avec la balance 20, permet au propriétaire de connaître l'identifiant de son animal, et d'accéder à des informations relatives à son animal sur le site du vétérinaire, ou à des informations spécifiques proposées sur le site de firmes pharmaceutiques ou de distributeurs de nourriture 34, grâce à cet identifiant.

Les firmes pharmaceutiques ou les distributeurs de nourriture 34 peuvent, via leur site Internet par exemple, communiquer directement à l'ordinateur du propriétaire des informations relatives à son animal, aux exercices, à l'alimentation, aux vaccins, etc, appropriés pour son animal.

Bien entendu l'exemple présenté concernant la mesure du poids d'un animal n'est pas limitatif. D'autres caractéristiques physiques ou physiologiques de l'animal peuvent être mesurées, comme sa taille, sa température, son rythme cardiaque, etc, et associées automatiquement à l'identifiant de l'animal, sans sortir du champ de protection de l'invention. Il est nécessaire pour ce faire que l'animal porte des moyens électroniques d'identification, ou puce électronique.

## Revendications

1. Système de gestion d'informations relatives à au moins un animal de compagnie portant des moyens électroniques d'identification (10) sous-cutanés, ledit système comprenant :
- des moyens de lecture (11) d'une information d'identification contenue dans lesdits moyens électroniques d'identification (10);
- des moyens de mesure (12) d'au moins une donnée physique associée audit animal ;
- des moyens de stockage et/ou transmission (13) de ladite au moins une donnée physique, en tenant compte de ladite information d'identification (10) ,
**caractérisé en ce que** lesdits moyens de mesure (12) comprennent au moins une balance (20) et **en ce que** lesdits moyens de lecture (11) sont intégrés à ladite balance (20).

2. Système de gestion d'informations selon la revendication 1, **caractérisé en ce que** lesdits moyens de lecture (11) comprennent au moins une antenne (23) mobile autour d'un axe de ladite balance (20).

3. Système de gestion d'informations selon l'une des revendications 1 et 2, **caractérisé en ce que** lesdits moyens de lecture (11) sont intégrés à un lecteur amovible.

4. Système de gestion d'informations selon l'une quelconque des revendications 1 à 3, **caractérisé en ce qu'**il comprend des moyens d'association d'une date à ladite au moins une donnée physique.

5. Système de gestion d'informations selon l'une quelconque des revendications 1 à 4, **caractérisé en ce qu'**il comprend au moins un processeur de traitement (32), permettant de relier ladite au moins une donnée physique à ladite information d'identification.

6. Système de gestion d'informations selon la revendication 5, **caractérisé en ce que** ledit processeur délivre une information de comparaison entre au moins une donnée physique mesurée précédemment et une donnée physique courante, associées audit animal.

7. Système de gestion d'informations selon la revendication 6, **caractérisé en ce que** ledit processeur délivre au moins une recommandation en fonction d'une analyse de ladite information de comparaison.

8. Système de gestion d'informations selon l'une quelconque des revendications 1 à 7, **caractérisé en ce qu'**il comprend au moins un haut-parleur.

9. Système de gestion d'informations selon l'une quelconque des revendications 1 à 8, **caractérisé en ce qu'**il comprend des moyens d'affichage et/ou de saisie d'informations liées audit animal.

10. Système de gestion d'informations selon la revendication 9, **caractérisé en ce que** lesdits moyens d'affichage et/ou de saisie comprennent au moins un élément appartenant au groupe comprenant :
- un écran d'affichage ;
- un écran tactile ;
- un clavier de programmation.

11. Système de gestion d'informations selon l'une quelconque des revendications 1 à 10, **caractérisé en ce que** lesdits moyens de stockage et/ou transmission (13) coopèrent avec au moins une connexion permettant un transfert d'au moins ladite donnée physique vers une unité externe.

12. Système de gestion d'informations selon la revendication 11, **caractérisé en ce que** ladite au moins une connexion appartient au groupe comprenant :
- une connexion USB (en anglais « Universal Serial Bus », en français « bus série universel) ;
- une connexion série ;
- une connexion Ethernet ;
- une connexion Bluetooth (marque déposée) ;
- une connexion Wifi.
